# EUROPÄISCHE PATENTANMELDUNG

(11) **EP 1 266 622 A1**
(43) Veröffentlichungstag der Anmeldung: **18.12.2002**
(21) Anmeldenummer: 02009560.0
(22) Anmeldetag: 26.04.2002
(51) Int. Cl.: A61B 8/00

(54) **Vorrichtung zur Verformungs- und/oder Bewegungserfassung**

(30) Priorität: 30.05.2001 DE 10126539
(71) Anmelder: friendly sensors AG, 07743 Jena (DE)
(72) Erfinder: Friedrichs, Arnd, Dr., 07743 Jena (DE); Kletzin, Ulf, Dr., 07745 Jena (DE)
(74) Vertreter: Behrmann, Niels, Dipl.-Ing.

(57) **Zusammenfassung**

Die Erfindung betrifft eine Vorrichtung zur Verformungs- und/oder Bewegungserfassung mit einem Paar von auf Ultraschallbasis zusammenwirkenden, bevorzugt piezoelektrisch wirkenden Sensoreinheiten und einer dem Paar von Sensoreinheiten nachgeschalteten elektronischen Messeinheit, die zum Erzeugen eines Verformungs- bzw. Bewegungssignals auf der Basis von einer Abstandsänderung zwischen dem Paar von Sensoreinheiten entsprechenden, elektronisch auswertbaren Signalen der Sensoreinheiten ausgebildet ist, wobei dem Paar von Sensoreinheiten eine Befestigungseinheit zum lösbaren Befestigen der Sensoreinheiten an einem Messobjekt, insbesondere an einem Körperteil einer Person, zugeordnet ist und das Paar von Sensoreinheiten durch eine ein elastisches und/oder deformierbares Abstandsmedium aufweisende Haltevorrichtung ultraschalleitend verbunden ist, die so ausgebildet ist, dass eine Deformation des Abstandsmediums zum Erzeugen des Verformungs- bzw. Bewegungssignals durch die Messeinheit detektierbar ist.

## Beschreibung

Die vorliegende Erfindung betrifft eine Vorrichtung zur Verformungs- und/oder Bewegungserfassung mittels eines Paares von Ultraschallsensoren, wobei die den Ultraschallsensoren nachgeschaltete elektronische Messeinheit das Verformungs- und/oder Bewegungssignal auf der Basis von Abstandsänderungen zwischen den Sensoreinheiten (entsprechend dann Laufzeitunterschieden des Signals zwischen den Sensoreinheiten) ermitteln kann.

Dabei sind aus dem Stand der Technik sowohl Verfahren bekannt, die die reine Laufzeit zwischen zwei Sensoren messen, als auch solche, welche ein von einem Sender reflektiertes Signal (Impuls-Echo-Methode) messen.

Insbesondere jedoch wenn eine solche bekannte Messung durch Luft oder andere Gase erfolgt, würde das Messsignal durch Reflexion an Grenzschichten, durch Temperaturänderungen, Umgebungsschall oder -- generell -- einen unbestimmten, weitgehend diffusen Signal- und Ausbreitungsverlauf in bzw. an einem Messobjekt nachteilig beeinflusst. Dies ist dann insbesondere kritisch, wenn der Ultraschall direkt in das zu untersuchende Medium eingekoppelt wird, was z. B. im Bereich der Werkstoffprüfung stattfindet. Die Problematik durch Ausbreitung in der Luft entsteht insbesondere dann, wenn das Paar von Sensoreinheiten als Sender bzw. Empfänger in einem definierten Abstand einander gegenüber angeordnet ist.

Ferner ist es aus dem Stand der Technik bekannt, Ultraschallsensoren im Zusammenhang mit bildgebenden Verfahren in der medizinischen Diagnostik zu verwenden, etwa im Bereich der Schwangerschafts- oder Organdiagnostik. Bei diesen Verfahren dient die Haut bzw. der menschliche Körper selbst als Übertragungsmedium. Dies gilt auch für solche Anwendungen, bei welchen, basierend auf Abstandsänderungen eines Paares von auf der Haut applizierten Ultraschallsensoren, eine Bewegungserfassung einer Person durchgeführt wird.

Hinzu kommt das generelle Problem, dass gerade im medizinischen Bereich auch der Eintragung von Ultraschallwellen in den menschlichen Körper mit großer Skepsis begegnet wird, so dass insbesondere bei topischen Messungen, d. h. solchen, für welche ein Eindringen von Ultraschall in einen Körper nicht erforderlich ist, der Bedarf nach Messverfahren wächst, bei denen sichergestellt werden kann, dass eine nachteilige Beaufschlagung des Körpers mit Ultraschallwellen nicht erfolgt.

Aufgabe der vorliegenden Erfindung ist es daher, eine auf Ultraschall-Abstandsmessung basierende Vorrichtung zur Verformungs- und/oder Bewegungserfassung dahingehend weiterzuentwickeln, dass diese nicht nur bei Einsatz im Zusammenhang mit einem menschlichen Körper, das Belasten des Körpers selbst mit dem Ultraschall vermeidet, sondern insbesondere auch die mit einem unmittelbaren Aufsetzen der Ultraschallsensoren auf dem Messobjekt, etwa der Haut einer Person, verbundenen Nachteile (speziell betreffend die damit verbundenen nachteiligen Beeinflussungen des Messsignals) vermieden werden können. Ferner ist es Aufgabe der vorliegenden Erfindung, eine bekannte Vorrichtung hinsichtlich der Universalität und Einfachheit ihrer Anwendung weiterzuentwickeln, wobei es insbesondere auch Aufgabe ist, einen vorbestimmten Abstand zwischen dem Paar von Sensoreinheiten reproduzierbar und unabhängig von einer jeweiligen zugrundeliegenden Oberfläche des Messobjekts sicherzustellen.

Die Aufgabe wird durch die Vorrichtung mit den Merkmalen des Hauptanspruchs gelöst; vorteilhafte Weiterbildungen der Erfindung sind in den Unteransprüchen beschrieben.

In erfindungsgemäß vorteilhafter Weise sind die Sensoreinheiten in einer Haltevorrichtung gehalten, welche selbst wiederum das erfindungsgemäße elastische bzw. deformierbare Abstandsmedium aufweist; über die erfindungsgemäße Befestigungseinheit ist zudem das Paar von Sensoreinheiten (bevorzugt über die Haltevorrichtung) auf dem betreffenden Messobjekt, also z. B. dem Körper einer Person, befestigbar.

Durch diese Vorgehensweise wird eine Reihe von Vorteilen gegenüber dem Stand der Technik realisiert: Nicht nur ermöglicht es die Haltevorrichtung, dass Paar von Sensoreinheiten in einem durch die Haltevorrichtung selbst klar definierten Abstand zu halten (unabhängig von einer Beschaffenheit einer unterliegenden Oberfläche des Messobjekts), auch ist durch die geeignete Wahl des Abstandsmediums der Haltevorrichtung (in einer bevorzugten Ausführungsform ist die Haltevorrichtung durch das Abstandsmedium selbst gebildet, d. h. aus dem das Medium bildenden Material geformt) ein eindeutiger Ausbreitungspfad in einem bekannten, ultraschall-leitfähigen Medium definiert, wodurch die Übertragungs- und mithin die Messgüte des erfindungsgemäßen Systems optimiert werden kann. Entsprechendes gilt für eine definierte bzw. durch geeignete Materialwahl definierbare Deformations- bzw. Verformungseigenschaften der durch die Haltevorrichtung bzw. das Abstandsmedium definierten Deformationsstrecke zwischen dem Paar von Sensoreinheiten: Hier bestehen nahezu beliebige Möglichkeiten, durch geeignete Materialwahl die Vorrichtung auf eine Vielzahl von möglichen Anwendungsfällen zuzuschneiden, wobei hohe Betriebssicherheit mit einfachster konstruktiver Realisierung und weitgehendem Schutz vor nachteiligen Umgebungs- und Umwelteinflüssen kombiniert werden kann.

Dies gilt insbesondere für eine bevorzugte Ausführungsform der vorliegenden Erfindung, wonach das Abstandsmedium aus nicht-biologischem Material, etwa Gummi oder Silikonkautschuk, gebildet und streifenförmig ausgeformt ist; an den Enden des jeweiligen Streifens sitzen (bevorzugt abnehmbar befestigt) die jeweiligen Sensoreinheiten, wobei in diesem Beispiel dann das Abstandsmedium selbst auch die Funktion der erfindungsgemäßen Haltevorrichtung übernimmt (alternativ wäre es z. B. vorstellbar, dass eine streifenförmige, flexible Kunststoffhülle ein leicht deformierbares Gel als Abstandsmedium aufnimmt). Ein derartiger Streifen lässt sich besonders geeignet mittels der weiterbildungsgemäß vorgesehenen Klebe- bzw. Haftfläche auf einem unterliegenden Messobjekt befestigen, wodurch dann Bewegungen der Messobjektoberfläche sich auf das Abstandsmedium bzw. die Haltevorrichtunng übertragen, dort für eine Deformation sorgen, welche sich in einer Abstandsänderung der Sensoreinheit niederschlägt und entsprechend als Bewegungs- bzw. Verformungssignal durch die Messeinheit ausgewertet werden kann.

Es versteht sich von selbst, dass, in der Funktion etwa einem Dehnungsmessstreifen vergleichbar, mit dieser Ausführungsform der Erfindung zahlreiche Anwendungsbeispiele, vom medizinisch-diagnostischen bis hin zum Fahrzeug- oder Gebäudebereich, abgedeckt werden können und lediglich die physischen Dimensionen und Verformungsparameter der Haltevorrichtung bzw. des Abstandsmediums geeignet an den Aufgabenzweck anzupassen sind.

Technisch hat es sich als besonders bevorzugt erwiesen, die Sensoreinheit als Piezosensoren zu realisieren und eine Radialschwingung eines solchen Sensors in das Übertragungsmedium einzukoppeln. Diese Vorgehensweise ermöglicht es, die erfindungsgemäße Vorrichtung besonders flach zu gestalten und damit den praktischen Anwendungsbereich zusätzlich zu erweitern.

Um die Möglichkeit eines Eintritts von Ultraschallwellen in das Messobjekt selbst weiter zu verringern, ist es weiterbildungsgemäß vorgesehen, die Haltevorrichtung mit einer Dämpfungs- und/oder Reflexionsschicht zu versehen, welche zwischen Sensoreinheit und der Oberfläche des Messobjektes liegt; typischerweise könnte dies eine Luftkammer bzw. ein vergleichbarer Hohlraum in der Haltevorrichtung sein, welcher Ultraschallwellen fast vollständig reflektiert. Neben der beabsichtigten weiteren Verminderung des Eintretens von Ultraschallwellen in das unterliegende Messobjekt (die Ultraschallausbreitung erfolgt ohnehin bevorzugt entlang des speziell angepassten Abstandsmediums) ergibt sich der Effekt einer weiteren Verbesserung der Signalgüte durch Unterdrückung unerwünschter Reflexionen vom Messobjekt selbst.

Je nach Anwendungsbereich bietet es sich zudem an, die Messeinheit als Bestandteil einer portablen, insbesondere batteriebetriebenen, Einheit auszubilden, die bevorzugt mittels einer drahtlosen Datenverbindung zur Übertragung eines geeigneten Auswertesignals und/oder weiterer Signale mit einer stationären Basiseinheit verbindbar ist. Aus dem Stand der Technik sind zahlreiche Vorgehensweisen bekannt, effizient, mit hoher Zuverlässigkeit und mit vergleichsweise geringem Aufwand hier für eine drahtlose Übertragung von Signalen zu sorgen, so dass insbesondere auch bei einer Verwendung der vorliegenden Erfindung im Bereich der Messung von Bewegungen an einer menschlichen Körperoberfläche hier die betreffende Person kaum in ihrer Bewegungsfreiheit beeinträchtigt ist.

Im Rahmen der vorliegenden Erfindung ist dabei die Begriffswahl "Paar von Sensoreinheiten" so zu verstehen, dass, über zwei aufeinander bezogene bzw. als Sender bzw. Empfänger zusammenarbeitende Sensoreinheiten hinaus, hier auch zusätzliche Paare von Sensoreinheiten im Rahmen eines Gesamtsystems zusammenwirken und gemeinsam ausgewertet werden können; auch ist als "Paar" insoweit eine Anordnung zu verstehen, bei welcher eine (aktive) Sensoreinheit sowohl Sender- als Empfängerfunktion wahrnimmt, während eine (oder mehrere) jeweils andere von Sensoreinheiten als -- definierte -- Reflektoren am anderen Ende des durch das Abstandsmedium definierten Übertragungskanals agieren und insoweit zwar selbst nicht Ultraschallwellen ausstrahlen oder empfangen, jedoch definiert das Sendesignal zur elektronischen Auswertung zurückleiten. Sowohl hinsichtlich der Anzahl von Reflektoren, als auch von Sende-/Empfangselementen sind hier, je nach Anwendungsfall, keine Grenzen gesetzt.

Neben den beanspruchten Verwendungen wird offensichtlich, dass die vorliegende Erfindung sich z. B. insbesondere auch im Bereich der Behindertentechnik bzw. der Rehabilitation eignet. Geeignet etwa auf bzw. an einem Körper einer behinderten Person appliziert, ermöglicht es die vorliegende Erfindung, dass der Behinderte im Rahmen der ihm zur Verfügung stehenden Motorik vorbestimmte Steuerungszwecke ausführen kann, welche dann in entsprechende Funktionen und Wirkungen umgesetzt werden können.

Betont werden sollte auch die prinzipielle Eignung der vorliegenden Erfindung, insbesondere in der bevorzugten Realisierung als streifenförmige Anordnung, als Dehnungsmessstreifen: Gerade im Zusammenhang mit Flugobjekten, bei welchen etwa eine Materialbelastung durch Lasten und Manöver besonders kritisch ist (z. B. das Beispiel des Cargolifter mit der teilweise kritischen Verformung an verschiedensten Stellen des Rumpfes bei Last) verdeutlicht einen sinnvollen praktischen Einsatzbereich der vorliegenden Erfindung. Selbiges gilt für Schwermaschinen, etwa Lastenkräne, Lastenregale usw., sowie die permanente Überwachung auf Verformung bzw. Materialbeanspruchung.

Alternativ bietet es sich an, die vorliegende Erfindung auch auf Mikrostrukturen anzuwenden, und dort ebenso zur Erfassung von Bewegungs-, Schallzuständen sowie Verformungen.

Die vorteilhafte Möglichkeit, das Paar von Sensoreinheiten in die Haltevorrichtung (die bevorzugt aus dem Abstandsmedium selbst besteht) einzubetten, sorgt zudem dafür, dass auf diesem Wege eine vorteilhaft gegen Umwelteinflüsse, etwa Feuchtigkeit und Verschmutzung, geschützte Anordnung geschaffen werden kann, so dass hier zusätzlich Potential für beliebige Einsetzbarkeit besteht. In der praktischen Realisierung sind Ausführungsbeispiele möglich, die einen Betrieb der vorliegenden Erfindung unter Wasser vorsehen.

Im Ergebnis ermöglicht es die vorliegende Erfindung, in überraschend einfacher und eleganter Weise nahezu beliebige Bewegungen, Krafteinflüsse, Betätigungs-, Regel-, Stellund Schaltbewegungen sowie Gewichtsbelastungen zuverlässig und mit geringem Aufwand zu detektieren und weiteren elektronischen Auswertungen zuzuführen. Wie sich aus den nachfolgend im Detail zu beschreibenden und auch in den Ansprüchen angegebenen Verwendungen ergibt, ist der Anwendungsbereich der vorliegenden Erfindung weit gesteckt und bietet die Möglichkeit, die vorliegende Technologie vorteilhaft für eine Vielzahl von Erfassungssituationen im alltäglichen sowie industriellen Umfeld zu nutzen, ohne dass durch die erfindungsgemäße Vorgehensweise Gefährdungen oder übermäßige Belastungen ausgehen.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Fig. 1:: eine schematische Ansicht der erfindungsgemäßen Vorrichtung zum Erzeugen von Meßdaten mit einem einzelnen Paar von Sensoreinheiten und einem ultraschallleitenden, dehnbaren Abstandsmedium sowie nachgeschalteten Funktionseinheiten;
- Fig. 2:: eine schematische Ansicht der Applikation der Abstandsmessvorrichtung an einem Kleidungsstück (20). Nach Anziehen des Kleidungsstücks ist die Länge L1 in Bezug auf L0 vorgedehnt (L1>L0);

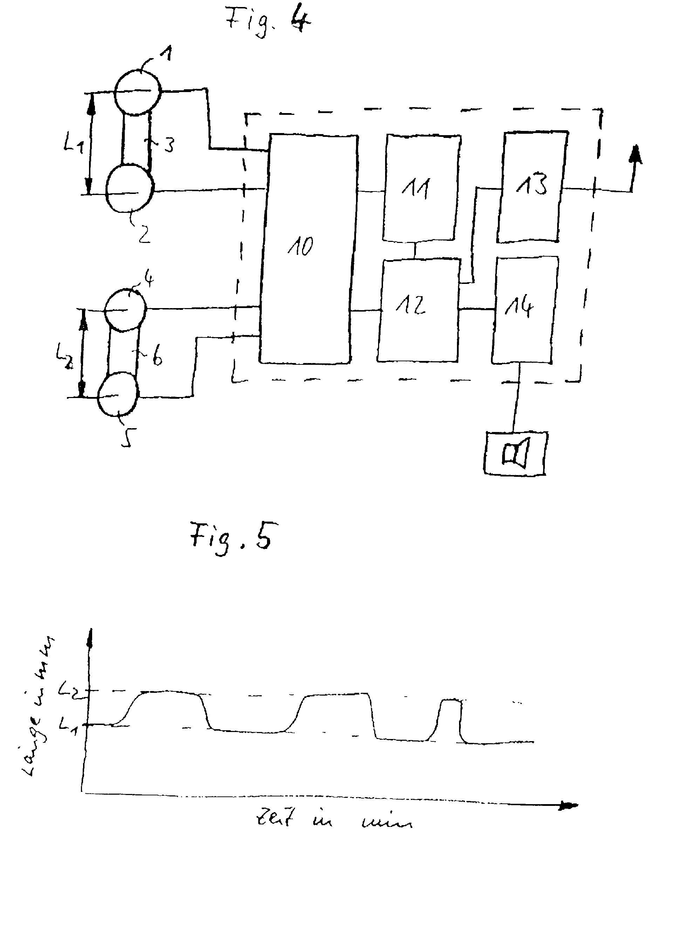

- Fig. 3:: eine Verdeutlichung der Verschiebung der Positionen und Abstände der Sensoren von Fig.1 als Reaktion auf eine Körperbewegung;
- Fig. 4:: eine alternative Ausführungsform mit 2 Paaren von Sensoreinheiten sowie einer nachgeschalteten portablen Meß- und Übertragungsvorrichtung;
- Fig. 5:: ein beispielhaftes Signal-Zeitdiagramm des in Fig.2 dargestellten Sensorpaares bei Körperbewegungen (vgl. Fig.3);

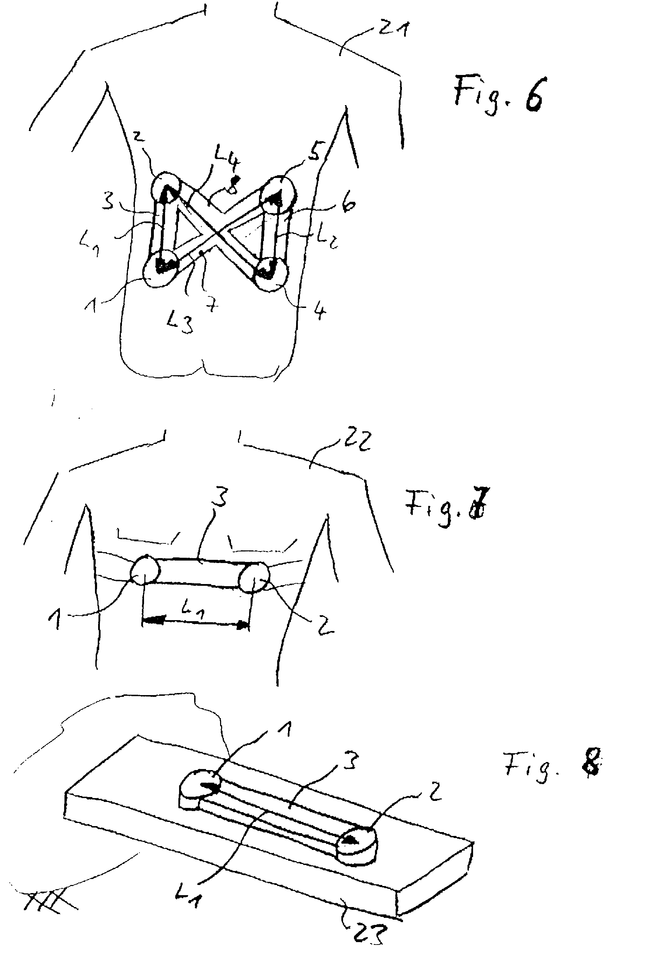

- Fig. 6:: beispielhafte Konfiguration einer Mehrzahl von Sensorpaaren und Abstandsmedien auf einem Shirt oder direkt auf der Haut (21) zur Erfassung und Auflösung komplexer Körperbewegungen;

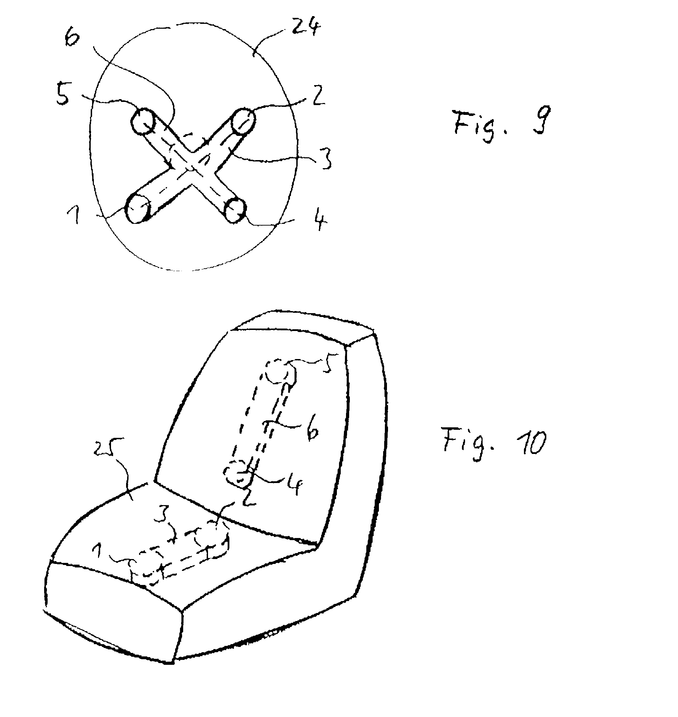

- Fig. 7:: beispielhafte Applikation eines Ultraschall-Abstandsmeßstreifens auf einem Brustgurt (22) zum Zweck der Messung von Vitalparametern (z.B. Atmung, Herzschlag);
- Fig. 8:: beispielhafte Applikation eines Ultraschall-Abstandsmeßstreifens auf einem langen Balken (23) zum Zweck der Messung der Verformung des Balkens unter Lasteinwirkung;
- Fig. 9:: beispielhafte Konfiguration von einer Einheit aus 2 Ultraschall-Abstandsmeßstreifen auf der Bauchdecke einer Schwangeren (24) zum Zweck der Wehenmessung;

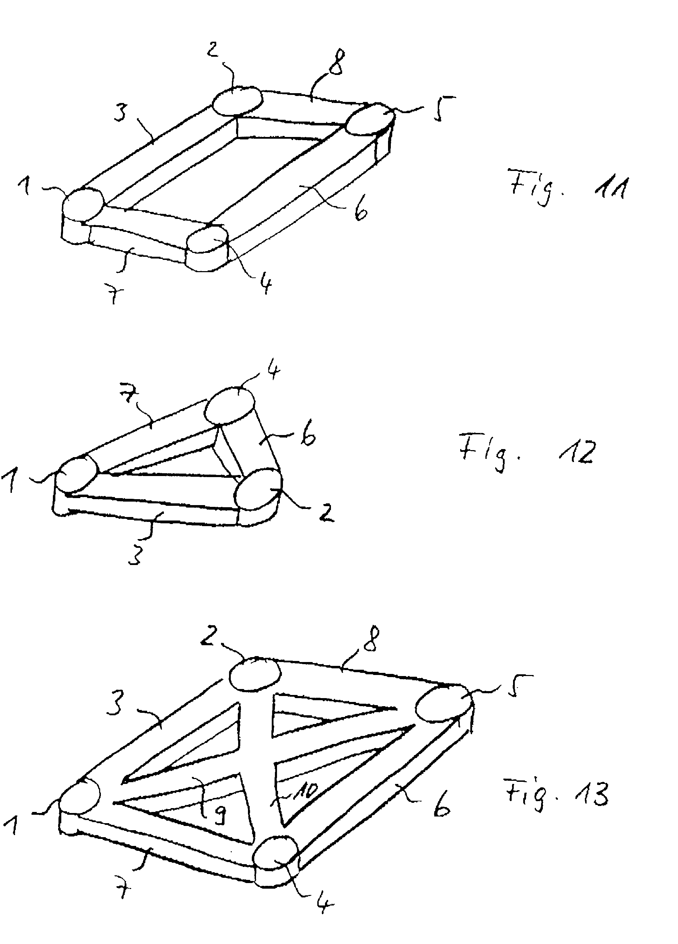

- Fig. 10:: beispielhafte Applikation zweier Ultraschall-Abstandsmeßstreifens in der Sitzfläche und der Lehne eines PKW-Sitzes (25) zum Zweck der Sitzbelegungserkennung und Fahrerpositionserkennung;

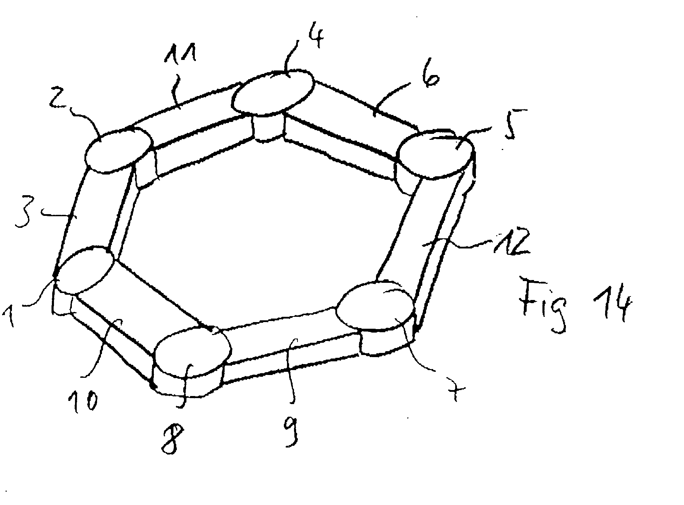

- Fig.11-14:: beispielhafte Konfigurationen der Anordnung einer Mehrzahl von Ultraschall-Abstandsmeßstreifen zur Messung komplexer Bewegungs- oder Verformungszustände. Denkbar sind auch Konfigurationen aus diesen und den in den Fig. 1, 6 und 9 dargestellten Anordnungen.

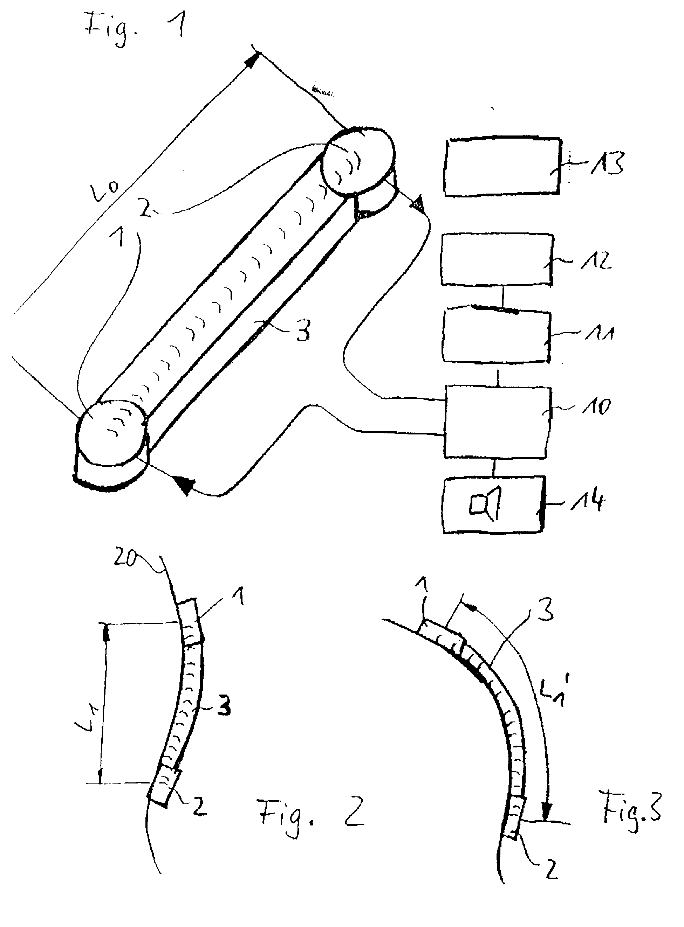

Die Fig. 1 zeigt eine Vorrichtung zum Erzeugen von Messdaten betreffend Bewegungen und/ oder Vitalparametern einer Person, oder Verformungen von Bauteilen, Baugruppen gemäss einer Ausführungsform der Erfindung mit mindestens einem Paar von Sensoreinheiten (1, 2), die in einen Abstand L1 voneinander angeordnet und durch ein ultraschallleitendes, dehnbares Abstandsmedium (3) miteinander verbunden sind. Dieses Abstandsmedium kann beispielsweise aus Gummi, Gel oder ähnlichem ausgeführt sein und besteht im vorliegenden Beispiel aus Silikonkautschuk.

Die Vorrichtung kann unmittelbar oder mittelbar derartig auf dem Messobjekt appliziert werden, dass die Sensoreinheiten (1, 2) in fester Beziehung zur Applikationsstelle stehen, sich ihr Abstand entsprechend den Bewegungen oder Verformungen des Messobjektes ändert und das Abstandsmedium (3) entsprechend gedehnt wird.

Der Sensoreinheit der vorliegenden Erfindung sind Sender und Empfänger einer Ultraschall-Entfernungsmeßeinheit zugeordnet, die in ansonsten bekannter Weise auf der Basis der durch Körperbewegungen/ Haltungsänderungen der Person oder Verformungen der Bauteile/ Baugruppen hervorgerufenen Laufzeitunterschiede des Ultraschallsignals innerhalb des entsprechend gedehnten Abstandsmediums, ein entsprechendes zur weiteren Auswertung geeignetes Signal erzeugt.

Mit dieser Vorrichtung steht ein Verfahren zur Erfassung von Körperbewegungen oder Bauteilverformungen zur Verfügung, dass jedoch nicht direkt auf der Haut appliziert werden muss, womit die Haut nicht als Übertragungsmedium für den Ultraschall wirkt.

Der Vorrichtung zugeordnet ist eine Entfernungsmesseinheit (10), die zur Erfassung elektrisch auswertbarer Signale der Einheit aus Sensoren und Abstandsmedium ausgebildet ist und ein erstes Abstandssignal sowie dessen Änderung erzeugt.

Der Entfernungsmesseinheit nachgeschaltet ist eine Speichereinheit (11), die zur Datenspeicherung der mit einer festen oder einstellbaren Abtastrate aktualisierten Abstandssignale dient, die zur weiteren Bearbeitung oder Anzeige mittels einer Ausgabeeinheit (14) ausgegeben werden können.

Die Vorrichtung umfasst weiterhin eine Auswerteeinheit (12), die die Erfassung bestimmter Signalformen oder Signalformänderungen ermöglicht, wie sie z.B. für die Beschreibung von Vitalparametern einer Person charakteristisch sind.

Weiterhin ist der Vorrichtung eine Kommunikationseinheit (13) zugeordnet, die die bedrahtete oder drahtlose Anbindung der Vorrichtung an eine zusätzliche Basisstation zur Überwachung und Auswertung der Messdaten ermöglicht.

Die in Fig. 1 dargestellten nachgeschalteten Einheiten (10 bis 14) können in die Sensoreinheit (1, 2) und/ oder das Abstandsmedium (3) portabel integriert sein oder alternativ als nachgeschaltete, getrennte Meß- und Übertragungsvorrichtung ausgeführt sein.

Die Fig. 1 zeigt schematisch die Applikation der, im einfachsten Ausführungsfall aus lediglich einem Paar von Sensoreinheiten und einem Abstandsmedium, bestehenden Vorrichtung; die weiteren Figuren verdeutlichen Varianten mit mehreren Paaren von Sensoreinheiten im Rahmen der Erfindung.

In Fig. 2 ist diese Vorrichtung beispielhaft auf der Kleidung einer Person appliziert. Das Kleidungsstück (20) ist enganliegend und folgt den Körperbewegungen bzw. Haltungsänderungen der Person dergestalt, dass sich der Sensorabstand L1 entsprechend der Hautverschiebungen ändert, da das Abstandsmedium zusammen mit dem Kleidungsstück in entsprechender Weise gedehnt wird. Die Vorrichtung wird im vorgespannten Zustand (L1 > L0) an der Kleidung befestigt, um so auch Abstandsverringerungen (L1' < L1) unter der Voraussetzung (L1' > L0) erfassen zu können.

Die Vorteile der Vorrichtung Verfahrens im Vergleich zum Stand der Technik sind evident:
- Kein Hautkontakt erforderlich
- Keine Einstrahlung von Ultraschallintensität in den Körper
- Keine Ankoppelprobleme (Übergangswiderstand)
- Einfache Befestigung (Detektionsmöglichkeiten in Klammer):
   - Einarbeiten in Gurt (Atmung, Körperhaltung, ...),
   - In Kleidungsstück(Atmung, Körperhaltung, Wehen, Schulter- und Ellenbogengelenkwinkel, ...)
   - in Hose (Beinwinkel, Kniewinkel, ...)
   - in Schuhe (Schrittzähler, ...)
   - in Handschuhe (Gelenkwinkel, ...)
   - Befestigung mittels Klettband o.ä. an Kleidungsstücken
   - Aufkleben, Annähen o.ä. auf / an Bauteile,
   - etc.
- Messung großer Verformungen auf ausgedehnten Bauteilen /Baugruppen

Weitere Einsatzmöglichkeiten sind in den Figuren dargestellt; die verschiedenen Ausführungsformen der Erfindung sind dazu geeignet, exemplarisch folgende Parameter zu erfassen:
- Körperhaltung
- Wehen
- Motiontracking (Gelenkbewegungen, etc.)
- Sitzbelegung
- Verformungsverhalten von Bauteilen und -gruppen (z.B. Leichtbau, Tragflächen, usw.)
- Atmung

Mit weiteren Ausführungsformen, die nicht durch Figuren verdeutlicht sind ist die Erfassung von etwa den folgenden Parametern möglich:
- Schrittfrequenzen, Trittfrequenzen, Schaltzuständen von Tastern und Schaltern (ein- und mehrstufig),
- Eingabegeräte,
- Biofeedbackgeräte,
- etc.

## Patentansprüche

1. Vorrichtung zur Verformungs- und/oder Bewegungserfassung mit
einem Paar von auf Ultraschallbasis zusammenwirkenden, bevorzugt piezoelektrisch wirkenden Sensoreinheiten
und einer dem Paar von Sensoreinheiten nachgeschalteten elektronischen Messeinheit, die zum Erzeugen eines Verformungs- bzw. Bewegungssignals auf der Basis von einer Abstandsänderung zwischen dem Paar von Sensoreinheiten entsprechenden, elektronisch auswertbaren Signalen der Sensoreinheiten ausgebildet ist,
**dadurch gekennzeichnet,**
**dass** dem Paar von Sensoreinheiten eine Befestigungseinheit zum lösbaren Befestigen der Sensoreinheiten an einem Messobjekt, insbesondere an einem Körperteil einer Person, zugeordnet ist und das Paar von Sensoreinheiten durch eine ein elastisches und/oder deformierbares Abstandsmedium aufweisende Haltevorrichtung ultraschalleitend verbunden ist, die so ausgebildet ist, dass eine Deformation des Abstandsmediums zum Erzeugen des Verformungs- bzw. Bewegungssignals durch die Messeinheit detektierbar ist.

2. Vorrichtung nach Anspruch 1, **dadurch gekennzeichnet, dass** das Abstandsmedium ein nicht-biologisches, insbesondere nicht-menschliches bzw. nicht-tierisches Material aufweist und insbesondere aus der Gruppe bestehend aus Gummi, Silikonkautschuk, Gel oder Mischungen von diesen ausgewählt ist.

3. Vorrichtung nach Anspruch 1 oder 2, **dadurch gekennzeichnet, dass** die das Abstandsmedium aufweisende Haltevorrichtung zum lösbaren oder unlösbaren Aufnehmen des Paares von Sensoreinheiten ausgebildet ist und/oder aus dem Abstandsmedium selbst besteht.

4. Vorrichtung nach einem der Ansprüche 1 bis 3, **dadurch gekennzeichnet, dass** die das Abstandsmedium aufweisende Haltevorrichtung streifenförmig ausgebildet ist und im Bereich jeweiliger Schmalseiten des Streifens das Paar von Sensoreinheiten hält.

5. Vorrichtung nach einem der Ansprüche 1 bis 4, **dadurch gekennzeichnet, dass** die Haltevorrichtung einen bevorzugt lösbaren Haft- und/oder Klebebereich als Befestigungseinheit aufweist, mit welchem die Vorrichtung auf dem Messobjekt bevorzugt abnehmbar befestigbar ist.

6. Vorrichtung nach einem der Ansprüche 1 bis 5, **dadurch gekennzeichnet, dass** eine des Paares von Sensoreinheiten als auf Piezobasis wirkender Ultraschallsensor und/oder Ultraschallempfänger ausgebildet ist.

7. Vorrichtung nach einem der Ansprüche 1 bis 6, **dadurch gekennzeichnet, dass** ein des Paares von Sensoreinheiten als passiv wirkender Reflektor für ein durch das Abstandsmedium geleitetes Signal der anderen des Paares von Sensoreinheiten ausgebildet ist.

8. Vorrichtung nach einem der Ansprüche 1 bis 8, **dadurch gekennzeichnet, dass** die Haltevorrichtung sowie die Sensoreinheiten zum Einkoppeln einer Radialschwingung einer insbesondere als Piezosensor ausgebildeten Sensoreinheit in das Abstandsmedium ausgebildet sind.

9. Vorrichtung nach einem der Ansprüche 1 bis 8, **gekennzeichnet durch** eine mindestens einer des Paares von Sensoreinheiten zugeordnete Dämpfungs- und/oder Reflexionsschicht zwischen Sensoreinheit und Messobjekt.

10. Verwendung der Vorrichtung nach einem der Ansprüche 1 bis 9 als auf bevorzugt manuelle Betätigung einer Bedienperson im Bereich der Haltevorrichtung reagierender/s Schalter, Regel- oder Stellglied.

11. Verwendung der Vorrichtung nach einem der Ansprüche 1 bis 9 zur Biege-, Zug-, Schubspannungs- und/oder Torsionserfassung an Fahrzeugen, Möbeln oder Gebäudeteilen.

12. Verwendung der Vorrichtung nach einem der Ansprüche 1 bis 9 zur Erfassung von Bewegungs- und anderen Körperparametern einer Person oder eines Tieres.

13. Verwendung der Vorrichtung nach einem der Ansprüche 1 bis 9 an bzw. unter einer Sitz- oder Liegefläche zur Belegungserfassung eines entsprechenden Sitzes bzw. einer Liege und/oder zur Bewegungserfassung einer Person auf der Sitz- oder Liegefläche.

14. Verwendung der Vorrichtung nach einem der Ansprüche 1 bis 9 auf einem Kleidungsstück bzw. als Teil eines Kleidungsstück zur Detektion einer Bewegung bzw. eines sich als Bewegung äußernden Körperparameters eines das Kleidungsstück tragenden Person.

15. Verwendung der Vorrichtung nach einem der Ansprüche 1 bis 9 mit einer Armbanduhr oder einem Armband, wobei die Haltevorrichtung als Armband wirkt kraftund/oder formschlüssig mit einem Körperbereich eines Trägers verbunden ist und zur Detektion herzschlagsbedingter Verformungen bzw. Bewegungen des Abstandsmediums in der Haltevorrichtung ausgebildet ist.

16. Verwendung der Vorrichtung nach einem der Ansprüche 1 bis 15 als Teil eines mit einem Kopfbereich in Kontakt stehenden Kopfgestells, insbesondere Brillengestells, wobei die Haltevorrichtung so in Kontakt mit dem Kopf bringbar ist, dass eine Bewegung eines Blutgefässes durch die Haltevorrichtung von der Kopfoberfläche detektierbar und durch die Messeinheit insbesondere als Herzschlagssignal auswertbar ist.
